# EUROPEAN PATENT APPLICATION

(11) **EP 0 922 465 A2**
(43) Date of publication of application: **16.06.1999**
(21) Application number: 98308846.9
(22) Date of filing: 28.10.1998
(51) Int. Cl.: A61M 16/04

(54) **Laryngeal mask assemblies**

(30) Priority: 02.12.1997 GB 9725389
(71) Applicant: Smiths Industries Public Limited Company, London, NW11 8DS (GB)
(72) Inventor: Pagan, Eric, Hythe, Kent CT21 6DN (GB)
(74) Representative: Flint, Jonathan McNeill

(57) **Abstract**

A laryngeal mask assembly has an inflatable cuff 13 mounted at the patient end 14 of a tube 1. The cuff 13 has a region 15 on its anterior surface 16 and a region 18 on its posterior surface 17 that expand preferentially when gas pressure is increased. The cuff 13 is inflated for insertion at low pressure such that the cuff takes one shape and is then further inflated when positioned correctly such that the region 15 on the anterior surface 16 expands and engages the epiglottis 5 and such that the region 18 on the posterior surface 17 expands and engages the adjacent tissue, pushing the anterior surface 16 forwardly.

## Description

This invention relates to laryngeal mask assemblies of the kind comprising a tube with a mask portion at its patient end, the tube having an opening into the centre of the anterior face of the mask portion.

It is common practice to use an airway known as a laryngeal mask for administering anaesthetic and ventilation gases to a patient. These airways comprise a tube with an inflatable mask or cuff at one end, the tube being inserted in the patient's mouth so that one end is located in the hypopharynx and so that the mask forms a seal in this region with the surrounding tissue. Laryngeal masks are described in, for example, US 5355879, US 5305743, US 5297547, US 5282464, GB 2267034, US 5249571, US 5241956, US 5303697, GB 2249959, GB 2111394, EP 448878, US 4995388, GB 2205499, GB 2128561, GB 2298797 and GB 2323290.

Laryngeal masks have several advantages over endotracheal tubes, which are longer and seal with the trachea below the vocal folds. One problem with laryngeal mask airways, however, is that it is difficult to provide the cuff, which is of relatively complex shape, at low cost.

It is an object of the present invention to provide an improved laryngeal mask assembly.

According to one aspect of the present invention there is provided a laryngeal mask assembly of the above-specified kind, characterised in that the mask portion includes an inflatable cuff arranged to take a first shape when filled with gas at low pressure and to take a second shape different from the first shape when the pressure of gas in the cuff is increased.

The second shape may include an enlarged portion on the anterior face of the cuff arranged to engage the epiglottis. The second shape may include an enlarged portion on the posterior face of the cuff arranged to engage adjacent tissue and push the anterior face of the cuff anteriorly. The cuff is preferably made by blow moulding. The cuff may have a reduced wall thickness in a region of the cuff, such that the region expands preferentially when gas pressure is increased. A region of the cuff may be treated to be weaker such that the region expands preferentially when gas pressure is increased. The cuff may have a region that is strengthened such that other regions expand preferentially when gas pressure is increased. The region that is strengthened may be strengthened by incorporating strengthening matter different from the cuff material. The region that is strengthened may be strengthened by increasing the thickness of the cuff material.

A laryngeal mask airway assembly according to the present invention, will now be described, by way of example, with reference to the accompanying drawings, in which:

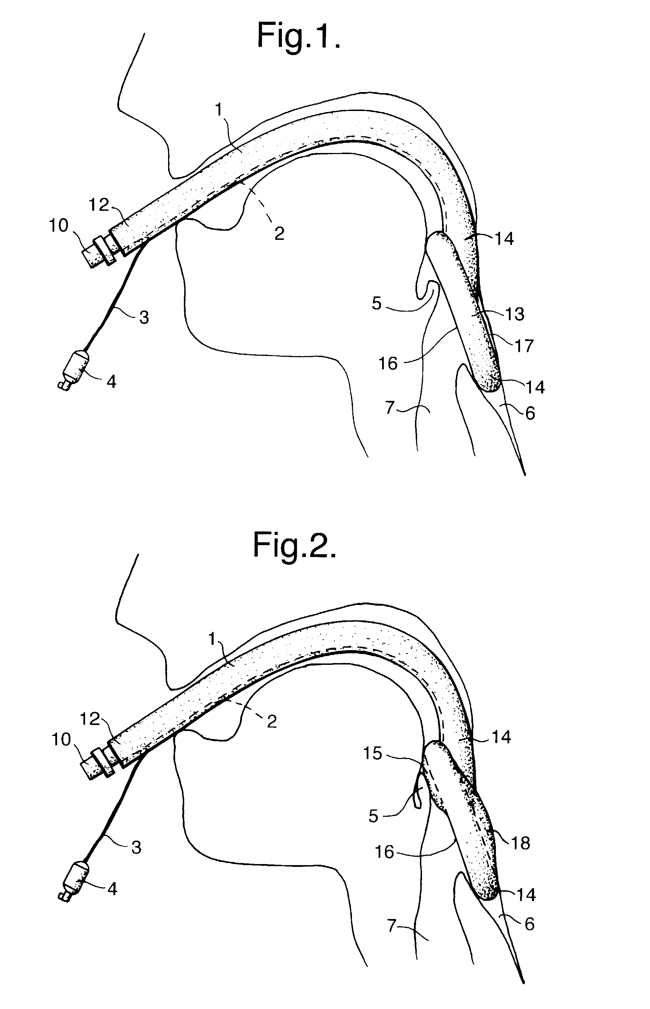

- Figure 1: is a side elevation view of the assembly with the cuff partially inflated;
- Figure 2: is a side elevation view of the assembly with the cuff fully inflated;

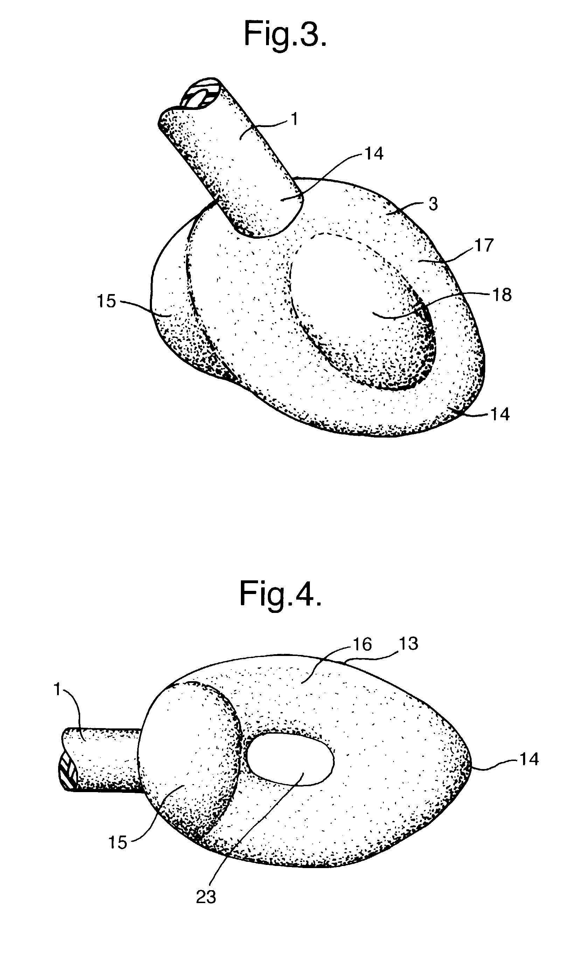

- Figure 3: is a perspective view of the posterior face of the patient end of the assembly with the cuff fully inflated; and
- Figure 4: is an end view of the anterior face of the patient end of the assembly with the cuff fully inflated.

The assembly comprises a bendable tube 1 of a plastics material, such as PVC, with a coupling 10 at its machine end 12. The tube 1 is curved along its length and has a mask portion with a cuff 13 attached at the patient end 14 of the tube.

The tube 1 is extruded with a small bore lumen 2 within its wall. The lumen 2 is connected towards the machine end of the assembly to an air line 3, which is terminated with a connector 4. The opposite, patient end of the lumen 2 opens into the cuff 13.

The cuff 13 is a ring or annulus of oval or elliptical shape, with a hollow centre 23 through which the tube 1 opens at the patient end of the assembly. The cuff 13 is of a plastics material such as PVC or polyurethane and is shaped so that it forms an effective seal with the pharynx or hypopharynx. The cuff 13 is made such that, when it is inflated to low pressure, with just sufficient air to fill the internal volume of the cuff, it takes a first shape, which is selected to be the best shape for introducing the cuff to the correct anatomical position. When additional air is supplied to the cuff 13, it changes to a second, different shape, which is the best shape for forming a seal in the pharynx and hypopharynx and for ensuring that the epiglottis 5 does not obstruct the opening 23 to the tube 1. This second, different shape is not simply an inflated, larger volume version of the first shape, and it is not simply a shape dictated by the limiting effect of the surrounding tissue, since the cuff 13 behaves in the same way when inflated outside the body, without the restrictions of the surrounding anatomy.

The first shape, used for insertion and positioning of the cuff 13 is relatively flat, enabling the forward end 14 of the cuff 13 to be located at the top of the oesophagus 6 with the tube opening 23 on its anterior face 16 facing into the top of the trachea 7. When inflated to its second shape, the cuff 13 expands preferentially in a region 15 at the upper, machine end of its forward, anterior face 16, so as to displace the epiglottis 5 anteriorly away from the opening to the tube. The cuff 13 also expands along its rear, posterior surface 17 in a central region 18 to bear against the posterior surface of the hypopharnyx and thereby urge the forward surface 16 of the cuff into close sealing engagement with the adjacent tissue around the opening of the trachea 7.

The cuff 13 is preferably made by a blow-moulding process to the first shape. There are several ways in which the cuff 13 can be arranged to expand preferentially in the selected regions 15 and 18, which involve either making these regions more easily deformed by internal pressure, or by making the other regions less easily deformed. One way is to give the regions 15 and 18 that are to be deformed a reduced wall thickness or to treat these regions to make the material of the wall intrinsically weaker or a combination of both. The reduced wall thickness could be achieved by inflating the cuff before it is fully cured within a mould having a cavity of the second shape, whilst ensuring that the cuff is not stretched beyond its elastic limit in the regions 15 and 18. An alternative way is to strengthen the other regions such as by incorporating strengthening matter different from the cuff material, such as webs or filaments. The regions could be strengthened instead by coating to increase its strength or thickness, or by a combination of additional strengthening and increased thickness.

In use, the cuff 13 is partly inflated to its first shape outside the body and is inserted to the correct location, in the usual way. When correctly located, an additional measured volume of air is pumped into the cuff 13 via the connector 4 to displace the epiglottis 5 and to push the anterior face 16 of the cuff into effective sealing engagement with the tissue surrounding the opening of the trachea 7.

## Claims

1. A laryngeal mask assembly comprising a tube (1) with a mask portion at its patient end (14), the tube (1) having an opening (23) into the centre of an anterior face (16) of the mask portion, characterised in that the mask portion includes an inflatable cuff (13) arranged to take a first shape when filled with gas at low pressure and to take a second shape different from the first shape when the pressure of gas in the cuff (13) is increased.

2. A laryngeal mask assembly according to Claim 1, characterised in that the second shape includes an enlarged portion (15) on the anterior face (16) of the cuff (13) arranged to engage the epiglottis (5).

3. A laryngeal mask according to Claim 1 or 2, characterised in that the second shape includes an enlarged portion (18) on the posterior face (17) of the cuff (13) arranged to engage adjacent tissue and push the anterior face (16) of the cuff (13) anteriorly.

4. A laryngeal mask assembly according to any one of the preceding claims, characterised in that the cuff (13) is made by blow moulding.

5. A laryngeal mask assembly according to any one of the preceding claims, characterised in that the cuff (13) has a reduced wall thickness in a region (15, 18) of the cuff, such that the region (5, 18) expands preferentially when cuff pressure is increased.

6. A laryngeal mask assembly according to any one of the preceding claims, characterised in that a region (15, 18) of the cuff (13) is treated to be weaker such that the region (15, 18) expands preferentially when gas pressure is increased.

7. A laryngeal mask assembly according to any one of the preceding claims, characterised in that the cuff (13) has a region that is strengthened such that other regions expand preferentially when gas pressure is increased.

8. A laryngeal mask assembly according to Claim 7, characterised in that the region that is strengthened is strengthened by incorporating strengthening matter different from the cuff material.

9. A laryngeal mask assembly according to Claim 7 or 8, characterised in that the region that is strengthened is strengthened by increasing the thickness of the cuff material.
